(19) Europäisches Patentamt
European Patent Office
Office européen des brevets

(11) EP 0 649 828 B1

(12) **FASCICULE DE BREVET EUROPEEN**

(45) Date de publication et mention de la délivrance du brevet:
**27.08.1997 Bulletin 1997/35**

(51) Int Cl.[6]: **C07C 19/08**, C07C 17/395

(21) Numéro de dépôt: **94402266.4**

(22) Date de dépôt: **10.10.1994**

(54) **Procédé de purification du 1,1,1,2-tétrafluoroéthane**

Verfahren zur Reinigung von 1,1,1,2-Tetrafluorethan

Process for the purification of 1,1,1,2-tetrafluoroethane

(84) Etats contractants désignés:
**DE ES FR GB IT NL**

(30) Priorité: **26.10.1993 FR 9312734**

(43) Date de publication de la demande:
**26.04.1995 Bulletin 1995/17**

(73) Titulaire: **ELF ATOCHEM S.A.**
**92800 Puteaux (FR)**

(72) Inventeurs:
- **Cheminal, Bernard**
  **F-69510 Soucieu-en-Jarrest (FR)**
- **Lantz, André**
  **F-69390 Vernaison (FR)**
- **Lacroix, Eric**
  **F-69480 Amberieux d'Azergues (FR)**

(74) Mandataire: **Leboulenger, Jean**
**Elf Atochem S.A.,**
**Département Propriété Industrielle,**
**Cedex 42 - La Défense 10**
**92091 Paris la Défense (FR)**

(56) Documents cités:
**EP-A- 0 548 742**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 649 828 B1

Printed by Jouve, 75001 PARIS (FR)

## Description

La présente invention concerne le domaine des hydrocarbures fluorés et a plus particulièrement pour objet la purification du 1,1,1,2-tétrafluoroéthane.

Ce composé, connu dans le métier sous la désignation F134a, est notamment destiné à remplacer le dichlorodifluorométhane (F12) actuellement utilisé comme fluide frigorifique, mais suspecté de contribuer à l'affaiblissement de la couche d'ozone stratosphérique. Pour ce faire, le F134a doit satisfaire à des normes de qualité relativement à la présence d'impuretés a priori toxiques comme les oléfines chlorofluorées.

Or, l'une des synthèses industrielle du F134a consiste en une fluoration catalytique en phase gazeuse du trichloréthylène ou du 1-chloro-2,2,2-trifluoroéthane (F133a) qui sous-produit toujours des quantités variables de 1-chloro-2,2,-difluoroéthylène (F1122) qui, vu son point d'ébullition (-17,7°C), s'avère très difficile à éliminer complètement du F134a (pEb. = -26,5°C) par simple distillation, surtout sous pression.

Le F134a obtenu selon ce procédé ou selon d'autres procédés contient en général encore d'autres composés oléfiniques tels que des butènes ou des propènes fluorés. Les oléfines en $C_4$ telles que $CF_3CF = CF\text{-}CF_3$ (F1318), $CF_3CF = CHCF_3$ (F1327), $CF_3CH = CClCF_3$ (F1326) et $CF_3CH = CHCF_3$ (F1336) ne sont pas particulièrement gênantes car elles peuvent être séparées du F134a par distillation. Les F134a obtenus industriellement, et en particulier celui obtenu par fluoration en phase gazeuse du trichloroéthylène ou du F133a, contiennent en général des quantités plus ou moins importantes de polyfluoropropènes tels que $CF_3CH = CH_2$ (1243), $CF_3CF = CH_2$ (1234), $CF_3CF = CHF$ (1225) ou leurs isomères qui sont très difficiles à séparer du F134a par distillation car leurs points d'ébullition sont très proches de celui du 134a.

De nombreux procédés de purification du F134a, et en particulier d'élimination du F1122 dans le F134a, ont déjà été proposés. On peut ainsi citer :

- l'hydrogénation catalytique du F1122 et/ou d'autres oléfines fluorées (WO 9008750, JP 02273634, JP 04095037) ;
- l'adsorption des impuretés sur charbon actif (EP 389334) ou sur tamis moléculaire (US 4906796, JP 03072437, EP 503796, EP 511612, EP 526002) ;
- l'oxydation du F1122 par le permanganate de potassium aqueux (US 4129603).

Aucun de ces procédés n'est entièrement satisfaisant du point de vue industriel. Ainsi, le traitement au permanganate aqueux nécessite un séchage du F134a après la purification qui augmente considérablement le coût de ce traitement. L'adsorption physique sur charbon ou tamis moléculaire ne peut être envisagée industriellement que pour un traitement de finition car, compte-tenu des capacités d'adsorption des matériaux proposés, il paraît tout-à-fait inéconomique de traiter des produits contenant plus que quelques dizaines de ppm d'impuretés adsorbables. Par ailleurs, d'après les documents cités ci-dessus, ces techniques d'adsorption ne permettent d'éliminer que le F1122 et pas les oléfines en $C_3$ ou $C_4$. L'hydrogénation catalytique nécessite des installations spéciales (compatibles avec l'hydrogène) qui ne sont industriellement envisageables que si le F134a a lui-même déjà été obtenu selon un procédé d'hydrogénolyse.

D'autres procédés proposés, tels que la fluoration des oléfines par du fluor élémentaire (EP 548744), sont tout aussi inintéressants industriellement.

Plus intéressante est la technique décrite dans le brevet US 4158675 qui concerne un procédé d'épuration du F1122 consistant à faire réagir les gaz issus de la réaction principale :

$$\text{F133a} + \text{HF} \rightleftharpoons \text{F134a} + \text{HCl}$$

sans séparation de l'HCl, de l'HF ou du F133a non transformés, dans un second réacteur maintenu à une température plus basse que celle de la réaction principale. A partir d'un mélange gazeux dont la teneur en F1122, ramenée aux composés organiques, est de 5300 vpm (volume par million), le traitement en ligne à 160°C conduit à un taux de 7 vpm en F1122.

Dans ce procédé, l'impureté éliminée (F1122) est refluorée en F133a c'est-à-dire en un produit recyclable. Mais l'inconvénient majeur de ce procédé réside dans la nécessité d'avoir à traiter un débit de gaz important et donc d'aboutir à un volume réactionnel élevé, ce qui entraîne un investissement et un coût d'entretien prohibitifs. Par ailleurs, le procédé ne concerne aussi que l'élimination du F1122.

Pour éviter ces inconvénients, on a proposé de traiter en phase gazeuse, en l'absence d'acide chlorhydrique, un mélange gazeux de F134a brut et d'HF en présence d'un catalyseur de fluoration (JP 04321632, EP 548742 et demande FR 9209700). Durant ce traitement, l'acide fluorhydrique s'additionne sur le F1122 et certains autres oléfines (chloro)

fluorées telles que $CF_2 = CFH$ (F1123), $CF_3CH = CHCF_3$ (F1336) et les transforme en composés saturés plus faciles à séparer et/ou à recycler par distillation. Ce procédé est particulièrement élégant, mais on a malheureusement constaté que, si le F1122 est particulièrement facile à éliminer selon cette méthode, d'autres oléfines fluorées telles que, par exemple, les fluoropropènes F1243, 1234 et 1225, sont très peu réactives dans ces conditions et ne peuvent pas être entièrement éliminées selon ce procédé.

Pour éviter les inconvénients des techniques précitées, la présente invention propose un moyen particulièrement efficace et économique pour purifier un F134a brut contenant des impuretés insaturées.

Le procédé selon l'invention consiste à traiter en phase gazeuse, un mélange gazeux de F134a brut, d'HF et d'oxygène ou d'air à une température comprise entre 200 et 350°C et sous une pression allant de la pression atmosphérique jusqu'à 2,5 MPa, en présence d'un catalyseur de fluoration, le rapport molaire HF/F134a étant compris entre 0,05 et 0,5 et le rapport molaire $O_2$/F134a étant compris entre 0,001 et 0,1.

Dans un F134a brut, le taux d'impuretés oléfiniques peut varier entre 50 et 15000 ppm (0,005 à 1,5 %) par rapport au F134a et est le plus souvent compris entre 500 et 5000 ppm (0,05 à 0,5 %). A côté des oléfines (chloro)fluorées, le F134a brut peut également contenir des quantités variables d'autres composés comme, par exemple, le F133a (0 à 7 %), le 1,1,1-trifluoroéthane (F143a), le monochlorotrifluoroéthane (F124) et le pentafluoroéthane (F125) ; la présence de ces impuretés saturées ne nuit nullement à l'efficacité du procédé selon l'invention.

Parmi les impuretés oléfiniques (chloro)fluorées présentes dans le F134a brut, le F1122 est en général l'impureté la plus importante. Les éventuelles autres impuretés oléfiniques telles que les F1123, F1243, F1234, F1225, F1318, F1327, F1326 et F1336 sont soit inexistantes soit généralement présentes à des teneurs plus faibles (10 à 500 ppm) ; parmi ces dernières, les plus gênantes sont les différents F1243, F1234 et F1225.

Grâce à l'utilisation conjointe d'HF et d'$O_2$, le procédé selon l'invention permet de réduire de façon importante la plupart des oléfines (chloro)fluorées. Il permet de transformer non seulement le F1122, mais aussi les oléfines en $C_3$ (F1243, F1234, F1225, ...) particulièrement difficiles à éliminer par traitement avec de l'acide fluorhydrique seul.

Dans le procédé selon l'invention, les oléfines (chloro)fluorées telles que les F1122, 1123, 1243, 1234, 1225, 1318, 1327, 1336,... peuvent réagir soit avec l'HF, soit avec l'oxygène. Le F1122 qui est l'impureté principale peut ainsi se transformer en F133a ou en produits d'oxydation (CO, $CO_2$, $COF_2$, ...). Le mécanisme de réaction de ces oléfines avec l'oxygène n'est pas entièrement connu, mais on peut supposer qu'elles réagissent en formant intermédiairement un époxyde qui peut se réarranger :

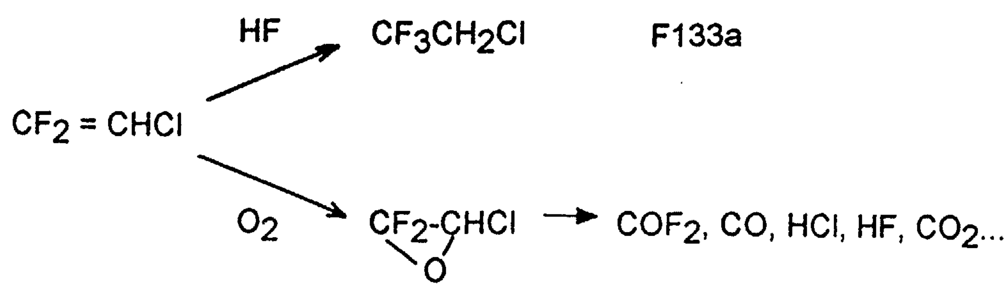


Le F133a étant directement recyclable au réacteur de fluoration, on a bien évidemment intérêt à privilégier la première réaction. Compte-tenu des différences de réactivité avec l'HF du F1122 et des autres oléfines présentes dans le F134a brut, il a pu être montré qu'un bon choix des conditions opératoires permet de minimiser la sous-production des produits d'oxydation du F1122 tout en transformant presque quantitativement aussi bien le F1122 que les autres oléfines comme les F1243, F1234 et F1225. La fluoration du F1122 en F133a permet ainsi de transformer cette impureté en un produit recyclable au réacteur mais cette prépondérance de la fluoration sur l'oxydation du F1122 ne limite pas l'invention car, dans le cas de faibles quantités de F1122, la perte par oxydation n'est pas importante.

Lors de la mise en oeuvre du procédé selon l'invention, l'oxygène (ou l'air) peut réagir avec le F134a ou avec certaines impuretés saturées telles que le F133a pour fournir des produits d'oxydation et de combustion de ces produits (CO, $CO_2$, $COF_2$,...). Bien que ces produits d'oxydation ne soient pas gênants et peuvent être facilement séparés du F134a, on a néanmoins intérêt à minimiser ces réactions secondaires car elles se traduisent par un perte de rendement.

En choisissant judicieusement les conditions opératoires (température, temps de contact, rapports molaires $O_2$/F134a et HF/F134a), il est possible de minimiser ces réactions de combustion du F134a ou des autres produits saturés éventuellement présents dans le F134a et de faire réagir l'oxygène principalement avec les impuretés oléfiniques. Le choix de la température est particulièrement important pour l'obtention d'une réaction sélective sans sous-production notable de produits d'oxydation du F134a ou éventuellement du F133a. Le choix du rapport molaire HF/F134a est tout aussi important car, en l'absence d'HF, l'élimination des oléfines est moins efficace et moins sélective. La dégradation du F134a par oxydation est dans ce cas nettement plus importante qu'en présence d'HF.

Le traitement catalytique en phase gazeuse du F134a brut avec de l'HF et de l'oxygène ou de l'air selon l'invention est avantageusement réalisé à une température comprise entre 200 et 350°C, de préférence entre 225 et 300°C, sous une pression comprise entre la pression atmosphérique et 2,5 MPa, de préférence entre la pression atmosphérique et 1,5 MPa.

Le temps de contact peut varier entre 10 et 200 secondes, mais on préfère un temps de contact compris entre 20 et 100 secondes.

Comme indiqué précédemment, le rapport molaire HF/F134a peut varier entre 0,05 et 0,5. On préfère cependant opérer avec un rapport molaire HF/F134a compris entre 0,125 et 0,200 et, plus particulièrement, un rapport molaire proche de celui correspondant à l'azéotrope HF-134a (0,15).

Le rapport molaire $O_2$/F134a peut varier entre 0,001 et 0,1, mais on préfère opérer avec un rapport molaire $O_2$/F134a compris entre 0,005 et 0,05.

A l'issue du traitement selon l'invention, le flux gazeux ne contient plus, ou seulement des traces, d'impuretés oléfiniques et peut alors être soumis aux opérations conventionnelles (lavage à l'eau, lavage avec une solution de soude-sulfite de sodium, séchage, distillation ...) pour séparer l'HF, l'oxygène ou l'air non transformés et les composés saturés autres que le F134a.

Si l'on désire minimiser les phénomènes d'entraînement du F134a par les gaz inertes tels que l'azote, on préférera généralement utiliser l'oxygène pur plutôt que l'air. Ce dernier peut cependant être préféré car il permet une mise en oeuvre du procédé plus facile.

Les catalyseurs de fluoration à utiliser pour la mise en oeuvre du procédé selon l'invention peuvent être des catalyseurs massiques ou des catalyseurs supportés, le support stable dans le milieu réactionnel étant, par exemple, un charbon actif, l'alumine, l'alumine partiellement fluorée, le fluorure d'aluminium ou le phosphate d'aluminium. Dans le cas du charbon, on évitera la combustion du support en prenant les précautions usuelles.

Parmi les catalyseurs massiques, on peut citer plus particulièrement l'oxyde de chrome préparé selon l'une quelconque des méthodes connues de l'homme du métier (procédé sol-gel, précipitation de l'hydroxyde à partir des sels de chrome, réduction de l'anhydride chromique, etc...). Les dérivés de métaux tels que nickel, fer, manganèse, cobalt, zinc, peuvent également convenir seuls ou en association avec le chrome, sous forme de catalyseurs massiques, mais aussi sous forme de catalyseurs supportés.

Les catalyseurs supportés peuvent être employés sous forme de billes, d'extrudés, de pastilles ou même, si l'on opère en lit fixe, sous forme de morceaux. Pour les catalyseurs massiques, la forme de pastilles ou de billes est généralement préférée. Lorsqu'on opère en lit fluide, on préfère utiliser un catalyseur sous forme de billes ou d'extrudés.

Comme exemples non limitatifs de catalyseurs, on peut mentionner :

- les microbilles d'oxyde de chrome obtenues par le procédé sol-gel comme décrit dans le brevet FR 2 501 062,
- les catalyseurs d'oxyde de chrome déposé sur charbon actif (brevet US 4 474 895), sur phosphate d'aluminium (brevet EP 55 958) ou sur fluorure d'aluminium (brevets US 4 579 974 et 4 579 976),
- les catalyseurs mixtes d'oxyde de chrome et de fluorure de nickel déposés sur fluorure d'aluminium (demande de brevet EP 0 486 333),
- les catalyseurs massiques à base d'oxydes de chrome et de nickel (demande de brevet EP 0546883).

Les brevets précités dont le contenu est incorporé ici par référence décrivent largement le mode de préparation de ces catalyseurs, mais aussi leur mode d'activation, c'est-à-dire de transformation préalable du catalyseur en espèces actives stables par fluoration au moyen d'HF gazeux dilué par des composés inertes (azote) ou non (air ou 1,1,2-trichloro-1,2,2-trifluoroéthane). Durant cette activation, les oxydes métalliques servant de matière active (par exemple l'oxyde de chrome) ou de support (par exemple l'alumine) peuvent être partiellement ou complètement transformés en fluorures correspondants.

Les exemples suivants illustrent l'invention sans la limiter. Sauf indication contraire, les teneurs (% et ppm) indiquées sont exprimées en volume.

**EXEMPLE 1 (Comparatif)**

Cet exemple est destiné à illustrer la réactivité des oléfines F1243, F1234 et F1225 avec de l'HF, sans oxygène, en présence d'un catalyseur de fluoration.

Dans un réacteur tubulaire en Inconel 600 de 28 mm de diamètre intérieur et d'un volume de 200 ml, on a placé 100 ml d'un catalyseur à base de fluorure de nickel et d'oxyde de chrome déposés sur fluorure d'aluminium. Les caractéristiques physico-chimiques de ce catalyseur, préparé comme décrit dans la demande de brevet EP 0 486 333 et activé en lit fixe par un mélange azote/HF, sont les suivantes :

- *Composition chimique(pondérale):*

- fluor : 58,6 %
- aluminium : 25,9 %
- nickel : 6,4 %
- chrome : 6,0 %
- oxygène : 3,1 %

- *Propriétés physiques*:

- densité apparente (en vrac) : 0,85 g/ml
- surface BET : 23 $m^2/g$
- volume des pores d'un rayon compris entre 4 nm et 63 µm : 0,4 ml/g
- surface des pores d'un rayon supérieur à 4 nm : 23 $m^2/g$

Après une ultime activation "in situ" du catalyseur à l'aide d'un mélange gazeux HF/F133a (rapport molaire : 0,4) entre 25 et 250°C, on a alimenté le réacteur avec un mélange gazeux constitué d'HF et de F134a brut dans des proportions telles que le rapport molaire HF/F134a soit égal à 0,2, c'est-à-dire proche de la composition azéotropique.

Le F134a utilisé contenait 50 ppm de F1243 + F1234 et 14 ppm de F1225. Les F1243 et F1234 n'étaient pas séparés dans les conditions d'analyse (chromatographie en phase gaz) et les 50 ppm correspondent à la somme F1243 + F1234. D'autres analyses du même mélange ont néanmoins montré que la teneur en F1243 était plus importante que celle de F1234.

La pression absolue du réacteur a été fixée à 1,2 MPa et le débit (d) d'alimentation du mélange F134a + HF a été ajusté de façon à voir un temps de contact (t) de 80 secondes, d et t étant liés par la relation :

$$t = \frac{3600 \text{ x } V \text{ x } 273 \text{ x } P \text{ x } 10}{22,4 \text{ x } d \text{ x } (T+273)}$$

où

P = pression en MPa
t = temps de contact en secondes
d = débit en moles/heure
V = volume de catalyseur en vrac, exprimé en litres
T = température du réacteur en degré Celsius

Un échantillon gazeux, débarrassé de l'HF en excès, était analysé par CPV en sortie de réacteur pour suivre l'évolution du taux d'oléfines dans les produits organiques.

Deux essais ont été réalisés respectivement à 250 et 300°C (durée 48 heures) et les résultats suivants ont été obtenus :

| Taux d'oléfines en sortie de réacteur (en ppm) | 250°C | 300°C |
|---|---|---|
| F1243 + F1234 | 30 | 20 |
| F1225 | 12 | 12 |

On a ensuite fait passer dans le même réacteur, sur le même catalyseur, dans les mêmes conditions de pression et de temps de contact, un mélange d'HF et de F134a (rapport molaire HF/F134a : 0,18); le F134a contenait 2400ppm de F1122. Comme ci-dessus deux essais ont été réalisés à 250°C et 300°C, et dans les deux cas, la teneur résiduelle en F1122 a été inférieure à 5 ppm.

Ces essais montrent ainsi très clairement que les oléfines F1234, F1243 et surtout F1225 sont nettement moins réactives vis-à-vis- de l'HF que le F1122 et qu'il est pratiquement impossible de les éliminer entièrement.

**<u>EXEMPLE 2</u> (Comparatif)**

Cet exemple, réalisé à pression atmosphérique avec un catalyseur analogue à celui de l'Exemple comparatif 1, est destiné à illustrer la mauvaise sélectivité de la réaction d'oxydation en l'absence d'HF.

Dans un réacteur en Inconel 600 de 40 mm de diamètre intérieur, on a placé 300 ml d'un catalyseur ayant la composition pondérale suivante :

- fluor : 56,0 %
- aluminium : 24,0 %
- nickel : 6,5 %
- chrome : 6,0 %
- oxygène : 7,5 %

Après activation du catalyseur avec un mélange $HF/N_2$, puis avec de l'HF pur à 360°C pendant une heure, on a procédé à un essai d'oxydation d'un F134a en l'absence d'HF dans les conditions suivantes :

| | |
|---|---|
| Température | 275°C |
| Pression | atmosphérique |
| Rapport molaire $O_2$/F134a | 0,01 |
| Temps de contact | 80 secondes |

Le F134a utilisé contenait 40 ppm de F1243 + F1234 et 4 ppm de F1225.

Après 25 heures de fonctionnement dans ces conditions, l'analyse des gaz, après lavage dans un barboteur à eau, a donné les résultats suivants :

- F1243 + F1234 : 13 ppm
- F1225 : 9 ppm
- F1123 ($CF_2$=CFH) formé : 14 ppm
- CO formé : 1 %
- $CO_2$ formé : 0,45 %

Ces résultats montrent clairement la grande sensibilité du F134a à l'oxydation en l'absence d'HF puisque les teneurs élevées en CO et $CO_2$ ne peuvent provenir que de l'oxydation du F134a. Ils montrent aussi qu'en l'absence d'HF l'élimination des oléfines est très difficile et qu'il s'est même formé du F1225 et du F1123.

## EXEMPLE 3

Dans le même réacteur et avec le même catalyseur qu'à l'exemple comparatif 2, on a fait passer un mélange HF/F134a/$O_2$ dans les conditions suivantes :

| | |
|---|---|
| Pression | atmosphérique |
| Temps de contact | 79 à 83 secondes |
| Rapport molaire HF/F134a | 0,2 à 0,5 |
| Rapport molaire $O_2$/F134a | 0,002 à 0,03 |
| Température | 275°C |

Le F134a contenait 40 ppm de F1243 + F1234 et 4 ppm de F1225.

Les résultats suivants ont été obtenus :

| RAPPORT MOLAIRE | | TAUX RESIDUELS D'OLEFINES (ppm) | | | |
|---|---|---|---|---|---|
| $\frac{O_2}{F134a}$ | $\frac{HF}{F134a}$ | F1243 + F1234 | F1225 | CO (%) | $CO_2$ (%) |
| 0,002 | 0,25 | 18 | < 1 | N.A.* | N.A.* |
| 0,005 | 0,30 | 8 | 3 | 0,09 | < 0,1 |
| 0,01 | 0,50 | 9 | 3 | < 0,1 | < 0,1 |
| 0,01 | 0,20 | < 1 | < 1 | 0,2 | 0,15 |
| 0,03 | 0,30 | 7 | 4 | 0,1 | < 0,1 |

* N.A. = non analysé

Comparés à l'Exemple 2, ces résultats illustrent clairement qu'en présence d'HF l'élimination des oléfines en $C_3$ est plus efficace, mais aussi plus sélective.

L'augmentation du rapport molaire HF/F134a se traduit par une diminution de la formation de CO et de $CO_2$ provenant essentiellement de l'oxydation du F134a.

**EXEMPLE 4**

Dans le même réacteur et avec le même catalyseur qu'à l'Exemple comparatif 1, on a fait passer un mélange de F134a-HF-air dans les conditions suivantes :

| | |
|---|---|
| Pression | 0,8 MPa |
| Temps de contact | 87 secondes |
| Rapport molaire HF/F134a | 0,2 |
| Rapport molaire air/F134a | 0,1 |
| Température | 275°C |

Le F134a utilisé contenait entre autres les impuretés suivantes :

| | | |
|---|---|---|
| F133a | : | 5 % |
| F124 | : | 0,8 % |
| F1122 | : | 2700 ppm |
| F1243 + F1234 | : | 35 ppm |
| F1225 | : | 11 ppm |

A la sortie du réacteur, les teneurs en oléfines étaient les suivantes :

| | |
|---|---|
| F1122 | 2 ppm |
| F1243 + F1234 | 8 ppm |
| F1225 | 4 ppm |

**EXEMPLE 5**

Dans le même réacteur et avec le même catalyseur qu'à l'exemple comparatif 1, on a fait passer un mélange HF/F134a/$O_2$ dans les conditions suivantes :

| | |
|---|---|
| Pression | 1,2 MPa |
| Temps de contact | 80 ou 180 secondes |
| Rapport molaire HF/F134a | 0,2 |
| Rapport molaire $O_2$/F134a | 0,002 à 0,01 |
| Température | 250 à 300°C |

Le F134a contenait 50 ppm de F1243 + F1234 et 14 ppm de F1225.
Les résultats suivants ont été obtenus :

| Température (°C) | Rapport molaire $O_2$/F134a | Taux résiduels d'oléfines (ppm) | | Taux d'oxydation du F134a | |
|---|---|---|---|---|---|
| | | F1243 + F1234 | F1225 | CO (%) | $CO_2$ (%) |
| 300 | 0,01 | 8 | 4 | 1 | 1 |
| 275 | 0,01 | 4 | 2 | 1 | 1 |
| 250 | 0,01 | 25 | 10 | 0,1 | 0,1 |
| 275 | 0,005 | 13 | 5 | 0,5 | 0,5 |
| 275 | 0,002 | 18 | 10 | 0,1 | 0,1 |
| 275* | 0,005 | 12 | 10 | 0,3 | 0,3 |

* Essai effectué avec un temps de contact de 180 secondes au lieu de 80 secondes pour les autres.

Les résultats de ces essais montrent que l'action simultanée de l'HF et de l'oxygène permet d'éliminer les oléfines F1243, F1234 et F1225 jusqu'à des valeurs très faibles. La température de 275°C constitue un optimum.

## EXEMPLE 6

Dans un réacteur tubulaire en Inconel 600 de 28 mm de diamètre intérieur et d'un volume de 200 ml, on a placé 50 ml d'un catalyseur constitué de microbilles d'oxyde de chrome massique, préparé comme décrit à l'exemple 3 du brevet FR 2 501 062. On a ensuite alimenté ce réacteur catalytique fonctionnant en lit fixe avec un mélange, à l'état gazeux, constitué de F134a brut, d'HF et d'oxygène dans des proportions telles que le rapport molaire HF/F134a soit égal à 0,20 et que le rapport molaire $O_2$/F134a soit égal à 0,01.

Le F134a brut contenait entre autres les impuretés suivantes :

| | |
|---|---|
| F124 | 2 % |
| F133a | 3,4 % |
| F1122 | 1370 ppm |
| F1243 + F1234 | 173 ppm |
| F1225 | 457 ppm |

La température du réacteur était fixée à 275°C et le débit d'alimentation du mélange a été réglé de façon à avoir un temps de contact de 80 secondes sous une pression de 1,2 MPa.

L'analyse du produit sorti du réacteur, après élimination de l'HF en excès, a foumi les résultats suivants :

| | |
|---|---|
| F1122 | 6 ppm |
| F1243 + F1234 | 35 ppm |
| F1225 | 12 ppm |

## Revendications

**1.** Procédé de purification d'un 1,1,1,2-tétrafluoroéthane (F134a) brut contenant des impuretés insaturées, caractérisé en ce que l'on traite en phase gazeuse, un mélange gazeux de 1,1,1,2-tétrafluoroéthane brut, d'acide fluorhydrique et d'oxygène ou d'air à une température comprise entre 200 et 350°C et sous une pression allant de la pression atmosphérique jusqu'à 2,5 MPa, en présence d'un catalyseur de fluoration, le rapport molaire HF/F134a étant compris entre 0,05 et 0,5 et le rapport molaire $O_2$/F134a étant compris entre 0,001 et 0,1.

**2.** Procédé selon la revendication 1 dans lequel les impuretés insaturées sont le 1-chloro-2,2-difluoroéthylène et/ou des oléfines (chloro)fluorées en $C_3$ ou $C_4$.

**3.** Procédé selon la revendication 1 ou 2, dans lequel on opère sous une pression comprise entre la pression atmosphérique et 1,5 MPa.

**4.** Procédé selon l'une des revendications 1 à 3, dans lequel le temps de contact est compris entre 10 et 200 secondes, de préférence entre 20 et 100 secondes.

**5.** Procédé selon l'une des revendications 1 à 4, dans lequel le rapport molaire HF/F134a est compris entre 0,125 et 0,200.

**6.** Procédé selon l'une des revendications 1 à 5, dans lequel le rapport molaire $O_2$/F134a est compris entre 0,005 et 0,05.

**7.** Procédé selon l'une des revendications 1 à 6, dans lequel on opère avec de l'oxygène pur.

**8.** Procédé selon l'une des revendications 1 à 6, dans lequel on utilise de l'air.

**9.** Procédé selon l'une des revendications 1 à 8, dans lequel le catalyseur de fluoration est un catalyseur massique ou supporté à base de chrome, nickel, fer, manganèse, cobalt et/ou zinc.

**Patentansprüche**

1. Verfahren zur Reinigung von Roh-1,1,1,2-tetrafluorethan (F134a), enthaltend ungesättigte Verunreinigungen, dadurch gekennzeichnet, daß man in der Gasphase eine gasförmige Mischung aus Roh-1,1,1,2-tetrafluorethan, Fluorwasserstoff und Sauerstoff oder Luft bei einer Temperatur zwischen 200 und 350 °C und unter einem Druck, der von Atmosphärendruck bis 2,5 MPa reicht, in Gegenwart eines Fluorierungskatalysators behandelt, wobei das HF/F134a-Molverhältnis zwischen 0,05 und 0,5 liegt und das $O_2$/F134a-Molverhältnis zwischen 0,001 und 0,1 liegt.

2. Verfahren nach Anspruch 1, bei dem die ungesättigten Verunreinigungen 1-Chlor-2,2-difluorethylen und/oder (chlor)fluorierte $C_3$-$C_4$-Olefine sind.

3. Verfahren nach Anspruch 1 oder 2, bei dem man unter einem Druck zwischen Atmosphärendruck und 1,5 MPa verfährt.

4. Verfahren nach einem der Ansprüche 1 bis 3, bei dem die Kontaktzeit zwischen 10 und 200 Sekunden, vorzugsweise zwischen 20 und 100 Sekunden, liegt.

5. Verfahren nach einem der Ansprüche 1 bis 4, bei dem das Molverhältnis von HF/F134a zwischen 0,125 und 0,200 liegt.

6. Verfahren nach einem der Ansprüche 1 bis 5, bei dem das Molverhältnis von $O_2$/F134a zwischen 0,005 und 0,05 liegt.

7. Verfahren nach einem der Ansprüche 1 bis 6, bei dem man mit reinem Sauerstoff verfährt.

8. Verfahren nach einem der Ansprüche 1 bis 6, bei dem man Luft verwendet.

9. Verfahren nach einem der Ansprüche 1 bis 8, bei dem der Fluorierungskatalysator ein Katalysator in Masse oder ein auf ein Trägermaterial aufgebrachter Katalysator auf Basis von Chrom, Nickel, Eisen, Mangan, Kobalt und/oder Zink ist.

**Claims**

1. Process for the purification of a crude 1,1,1,2-tetrafluoroethane (F134a) containing unsaturated impurities, characterized in that a gaseous mixture of crude 1,1,1,2-tetrafluoroethane, hydrofluoric acid and oxygen or air is treated in the gas phase, at a temperature between 200 and 350°C and at a pressure ranging from atmospheric pressure to 2.5 MPa, in the presence of a fluorination catalyst, the HF/F134a molar ratio being between 0.05 and 0.5 and the $O_2$/F134a molar ratio being between 0.001 and 0.1.

2. Process according to Claim 1, in which the unsaturated impurities are 1-chloro-2,2-difluoroethylene and/or $C_3$ or $C_4$ (chloro)fluorinated olefins.

3. Process according to Claim 1 or 2, in which the reaction is carried out at a pressure between atmospheric pressure and 1.5 MPa.

4. Process according to one of Claims 1 to 3, in which the contact time is between 10 and 200 seconds, preferably between 20 and 100 seconds.

5. Process according to one of Claims 1 to 4, in which the HF/F134a molar ratio is between 0.125 and 0.200.

6. Process according to one of Claims 1 to 5, in which the $O_2$/F134a molar ratio is between 0.005 and 0.05.

7. Process according to one of Claims 1 to 6, in which the reaction is carried out with pure oxygen.

8. Process according to one of Claims 1 to 6, in which air is used.

9. Process according to one of Claims 1 to 8, in which the fluorination catalyst is a bulk or supported catalyst based

on chromium, nickel, iron, manganese, cobalt and/or zinc.